# EUROPEAN PATENT SPECIFICATION

(11) **EP 2 202 202 B1**
(45) Date of publication and mention of the grant of the patent: **21.02.2018**
(21) Application number: 10160098.9
(22) Date of filing: 02.05.2006
(51) Int. Cl.: A61F 2/91, D01F 9/12, D01D 5/00, A61F 2/06, D04H 1/00, B32B 5/02, B32B 5/28, B32B 25/08, C04B 35/524, C04B 35/83, B29C 70/12, B32B 5/26, B32B 9/04, B32B 27/12, F41H 5/04, A61F 2/93, B32B 9/00, C08K 3/04, D21H 13/50, C01B 32/16, D01F 9/14, B32B 5/00, B32B 27/04, B82Y 30/00

(54) **Carbon nanotube composite materials**
Kohlenstoffnanorohrverbundmaterialien
Matériaux en composites de nanotubes en carbone

(30) Priority: 03.05.2005 US 677116 P; 20.01.2006 US 760748 P
(43) Date of publication of application: 30.06.2010
(62) Divisional of application: 06849762.7
(73) Proprietor: Nanocomp Technologies, Inc., Lebanon, NH 03766 (US)
(72) Inventor: Lashmore, David S., Lebanon, NH 03766 (US); Brown, Joseph J., Boulder, CO 80309 (US)
(74) Representative: CMS Cameron McKenna Nabarro Olswang LLP

(56) References cited:
- JP-A- 2004 315 297
- JP-A- 2005 075 672
- JP-A- 2005 281 672
- GOU J G: "Passage: Nanotube Bucky Papers and Nanocomposites - Single-Walled Carbon Nanotube Bucky Paper/Epoxy Composites: Molecular Dynamics Simulation and Process Development, Ph.D. Dissertation", DISSERTATION, MARBURG AN DER LAHN, 1 January 2002 (2002-01-01), pages 93-126, XP008097144,

## Description

### TECHNICAL FIELD

The present invention relates to carbon composites and methods of manufacturing same, and more particularly, to a carbon composite having a relatively high loading of carbon nanotubes.

### BACKGROUND ART

Carbon nanotubes are known to have extraordinary tensile strength, including high strain to failure and relatively high tensile modulus. Carbon nanotubes may also be highly resistant to fatigue, radiation damage, and heat. To this end, the addition of carbon nanotubes to composites can increase tensile strength and stiffness. Examples of composites that have incorporated nanotubes include epoxy-nanotube, Krayton-nanotube, PEEK (polyaryletherketone)-nanotube, phenyl formaldehyde-nanotube, RESOL- nanotube, furfuryl alcohol-nanotube, pitch-nanotube, latex-nanotube, polyethylene-nanotube, polyamide-nanotube, or carbon-carbon (nanotube) composites.

Unfortunately adding even a small amount of carbon nanotubes to, for instance, a resin matrix to subsequently generate the desired composite can increase the viscosity of the matrix significantly. As a result, a maximum of only between 1% to 5% by weight of carbon nanotubes may be added to a resin using current mixing technology.

Accordingly, it would be advantageous to provide a method for manufacturing a composite having a relatively high amount of carbon nanotubes, so that a composite with low density and high modulus and strength may be created. In addition, it would be advantageous to provide a carbon nanotube composite with such characteristics.

JP 2004 315297 describes a kneaded nano carbon composite material having a structure which includes a carbon nanotube and a resin.

Buckypapers, which are thin sheets made from an aggregate of carbon nanotubes, are described in a paper by J.G. Gou, entitled "Single-Walled Carbon Nanotube Bucky Paper/Epoxy Composites: Molecular Dynamics Simulation and Process Development", Ph.D. Dissertation, MARBURG AN DER LAHN, 1 January 2002 (2002-01-01), pages 93-126.

### SUMMARY OF THE INVENTION

The present invention provides a composite material having a mass having a thickness ranging from 0.01 mm to more than 3 mm. The composite also includes a plurality of non-woven nanotubes dispersed throughout the mass, such that a plurality of voids exists between the non-woven nanotubes. The mass is formed by a plurality of non-woven nanotube sheets bonded to one another. The composite further includes a resin material situated within the voids between the non-woven nanotubes to provide the mass with structural integrity. The channels may extend throughout the mass, the channels providing a pathway to permit fluid byproducts to escape during manufacturing of the composite material. In an embodiment, the amount of nanotubes that exist in the composite can be more than 5 % by volume of the mass. The nanotubes may include one of carbon nanotubes, silicon-carbon nanotubes, boron-carbon nanotubes, nitrogen- carbon nanotubes, or a combination thereof. Moreover, the resin material may differ in different areas of the mass so as to provide the mass with different properties in those areas. The mass may be capable of being formed into a three dimensional shape or structure. The three dimensional shape or structure may include molded high strength parts, including combat helmets, motorcycle helmets, football helmets and the like, parts for high temperature applications, including hypersonic parts and rocket nozzles, and biomedical devices and parts, including hear valves and stents.

A method is described for contextual purposes only in which a suitable catalyst may be added to a high-carbon-containing resin to generate an in situ composite having a glassy carbon matrix reinforced by a "grown-in" array of carbon nanotubes. The method includes initially providing a carbon-containing resin material. Next, an appropriate concentration of catalyst particles may be added to the carbon-containing resin material. In one embodiment, the concentration of the catalyst particles can be 0.005 percent to 5 percent by weight of catalyst particles to carbon in the resin material. Thereafter, the catalyzed resin may be placed in an inert atmosphere and subject to a temperature range of from 1000° C to 2000° C, at which point carbon in the resin to begins to couple to the catalyst particles. Continual attachment of carbon to the particles and subsequently to existing carbon on the particles can lead to the growth, within the resin material, of an array of carbon nanotubes and the formation of the composite material. In an embodiment, a sulfur containing compound may be added to the catalyzed resin to augment subsequent activities of the catalyst particles when the catalyzed resin is subject to high temperature. The resin material may include alkyl-phenyl formaldehyde. In the step of adding, the catalyst particles may include one of ferrocene; iron nano-particles; iron pentacarbonyl; nano- particles of magnetic transition metals or their alloys; oxides, nitrates or chlorides of these metals; any combination of the oxides with reducible salts; or organometallic compounds of these metals. The step of adding may include adding one of Nb, Mo, Cr, or a combination thereof to the catalyzed resin to refine the size of the catalyst particles, in order to control the size of the nanotubes being grown. The step of adding may include adding different catalyst particles in different areas of the resin material to subsequently provide different properties in is different areas of the resulting composite material. The step of subjecting may include placing the catalyzed resin into an inert atmosphere having argon, helium, or other inert gases. In some embodiments, in the step of subjecting, the temperature is 1700° C. The step of subjecting may include raising the temperature at a rate of from less than 1 degree C to 1 degree C per minute. The step of subjecting may include diffusing a fluid by-product from the resin. In the step of permitting, the attachment of carbon to an existing carbon on the catalyst particle may occur in series, so as to lead to the growth of a carbon nanotube from a catalyst particle. The method may further include subjecting the composite material to a final ramp temperature up to 3000°C. Another embodiment, which is not according to the present invention, provides a composite material having a glassy carbon matrix. The composite material also includes a plurality of catalyst particles dispersed throughout the matrix. The composite material further includes an array of nanotubes, each extending from a catalyst particle, so as to provide the glassy carbon matrix with added structural integrity. The catalyst particles, in an embodiment, can act as an x-ray contrasting agent, and to the extent the catalyst particles have magnetic properties, can act to provide magnetic properties to the composite. In addition, the amount of nanotubes that exist in the composite can be more than 5 % by weight of the mass. Moreover, the resin material may differ in different areas of the mass so as to provide the mass with different properties in those areas. The nanotubes may include one of carbon nanotubes, silicon-carbon, boron-carbon nanotubes, nitrogen-carbon nanotubes, or a combination thereof. The plurality of catalyst particles may be different in make up or concentration in different areas of the matrix, so as to provide the composite material with different properties in those areas. The plurality of catalyst particles may act as a contrasting agent. The glassy carbon matrix may be capable of being formed into a three dimensional shape or structure having the array of nanotubes therein. The glassy carbon matrix may be capable of being formed as a thin film or coating having the array of nanotubes therein. The glassy carbon matrix may be capable of being extruded into a filamentous fiber having the array of nanotubes therein. The filamentous fiber may have a diameter ranging from 0.5 microns to 500 microns. The glassy carbon matrix may be capable of being formed into molded high strength parts, including combat helmets motorcycle helmets, football helmets and the like; parts for high temperature applications, including hypersonic parts and rocket nozzles; biomedical devices and parts, including hear valves and stents; and sporting goods, such as rackets and golf clubs.

A stent for placement within a vessel may utilise the composite material. The stent, in an embodiment, includes a tubular expandable matrix having a plurality of intersecting filaments. The stent also includes a plurality of nanotubes situated within a core of each filament. In one embodiment, a glassy carbon material may be situated the nanotubes. The stent further includes a pathway extending from one end of the tubular matrix to an opposite end to permit fluid within the vessel to flow therethrough, and having a surface defined by the glassy carbon material. In an embodiment, a patterned surface may be provided the tubular matrix to permit the matrix to engage against a surface of the vessel, so as to minimize its movement within the vessel. The stent may further include a catalyst particle at an end of each nanotube, such that the particles can act as a contrasting agent. The catalyst particle at the end of each nanotube may provide the stent with magnetic properties.

Also for contextual purposes, a method for manufacturing a composite fiber is described, the method comprising: providing a carbon-containing resin material; adding an appropriate concentration of catalyst particles to the carbon-containing resin material; extruding the catalyzed resin material into a filamentous fiber at a temperature that permits polymerization of the filament; subjecting the extruded filamentous fiber to a temperature range of from 1000° C to 2000° C; allowing carbon in the resin to couple to the catalyst particles; and permitting subsequent growth, within the resin material, of an array of carbon nanotubes from the catalyst particles, so as to result in the formation of the composite fiber. In the step of extruding, the temperature may be at a range of from 50° C to 150° C. In the step of extruding, the fiber may have a diameter ranging from 0.5 microns to 500 microns.

In another method for manufacturing a composite, whereby at least one sheet of non-woven carbon nanotubes or nanofibers may be infiltrated with an appropriate resin. The method may comprise providing a sheet of non- woven nanotubes having voids between the nanotubes; infiltrating the voids between the nanotubes with a resin material; placing the infiltrated sheet into an inert atmosphere; and exposing the infiltrated sheet to a temperature range of from 1000° C to 2000° C to transform the infiltrated sheet into the composite material. In the step of providing, the nanotubes may be carbon nanotubes. The step of infiltrating may include coating the sheet with a fluid resin material. In the step of coating, the fluid resin material may be furfuryl alcohol. The step of infiltrating may include melting a sheet of a polymeric resin material onto the non-woven sheet. In the step of melting, the sheet of resin material may include one of RESOL resin, polyamide resin, epoxy resin, Krayton resin, polyethylene resin, polyaryletherketone resin, or a combination thereof. In the step of placing, the inert atmosphere may include argon, helium, or other inert gases. In the step of exposing, the temperature may be 1700° C. The step of exposing may include raising the temperature at a rate of from less than 1 degree to 1 degree C per minute. The step of exposing may include diffusing a fluid by-product from the sheet. The step of providing may include layering a plurality of non-woven sheets on one another. The step of infiltrating may include coating each non-woven sheet on a heated substrate, so that curing of the resin can occur before the next non-woven sheet can be layered thereonto. The step of infiltrating may include positioning a sheet of a polymeric resin between adjacent non-woven sheets. In the step of positioning, the sheet of polymeric resin between one pair of adjacent non-woven sheets may be different than the sheet of polymeric resin between another pair of adjacent non- woven sheets to provide different properties in different areas of the resulting composite material. The method may further include bonding the plurality of non-woven sheets to one another to provide a formed mass. The step of bonding may include applying heat to the layer of sheets at a temperature ranging from 125° C to 350° C. The method may further include subjecting the formed mass to a final ramp temperature up to 3000° C.

In an example, the method includes initially layering a plurality of non-woven sheets of carbon nanotubes. Next, a resin material may be applied to the non-woven sheets to infiltrate voids between the carbon nanotubes with a resin material. In an embodiment, each non-woven sheet may be coated with a resin material. Alternatively, a sheet of polymeric resin may be situated between adjacent non-woven sheets and melted into the voids. To the extent necessary, prior to infiltrating the voids with a resin material, a surface treatment process can be applied to the carbon nanotubes to facilitate bonding of the resin material to the nanotubes. The infiltrated sheets may thereafter be bonded with one another to provide a formed mass or structure. The infiltrated sheets may then be exposed to a temperature range of from 1000° C to 2000° C to transform the infiltrated sheets into the composite material.

### BRIEF DESCRIPTION OF DRAWINGS

Fig. 1 illustrates sheets of non-woven carbon nanotubes for use in the manufacture of a carbon-carbon composite in accordance with one embodiment of the present invention.
Fig. 2 illustrates sheets of non-woven carbon nanotubes for use in the manufacture of a carbon-carbon composite in accordance with another embodiment of the present invention.
Fig. 3 illustrates a glassy carbon matrix composite manufactured in accordance with another embodiment of the present invention.
Figs. 4A-B illustrate a stent made from a composite material of the present invention.
Figs. 5A-B illustrate various matrix or filament designs for use with the stent of the stent illustrated in Figs. 4A-B.
Fig. 6 is a cross-sectional view of a filament of the stent illustrated in Fig. 4.
Fig. 7A-B illustrate one patterned design for an exterior surface of the stent shown in Fig. 4 to permit anchoring of the stent within a vessel.
Fig. 8 illustrates other patterned designs for use in connection with the stent in Fig. 4.

### DESCRIPTION OF SPECIFIC EMBODIMENTS

Carbon nanotubes for use in connection with the present invention may be fabricated using a variety of approaches. Presently, there exist multiple processes and variations thereof for growing carbon nanotubes. These include: (1) Chemical Vapor Deposition (CVD), a common process that can occur at near ambient or at high pressures, (2) Arc Discharge, a high temperature process that can give rise to tubes having a high degree of perfection, and (3) Laser ablation.

At present, CVD appears to be one of the more attractive approaches from a commercial standpoint for fabricating carbon nanotubes. However, since growth temperatures for CVD can be comparatively low ranging, for instance, from 600° C to 1300° C, carbon nanotubes, both single wall (SWNT) or multiwall (MWNT), may be grown, in an embodiment, from nanostructural catalyst particles supplied by reagent carbon-containing gases (i.e., gaseous carbon source).

Examples of catalyst particles that may be used in connection with CVD include ferromagnetic transition metals, such as iron, cobalt, nickel, oxides, nitrates or chlorides of these metals. In certain instances, these catalyst particles may be combined with molybdenum or ceramic carriers or with each other. In the case of oxides, the oxides may be reduced to metallic form, as a result of the excess of hydrogen present in these reactions.

Suitable carbon-containing gases for the CVD process, in one embodiment, can include acetylene, methane, ethylene, ethanol vapor, methanol vapor and the like.

Although there exist a variety of CVD processes, an example of a CVD process that can be used in connection with the present invention is disclosed in U.S. Patent Application Publication US 2005/0170089, which application is hereby incorporated herein by reference.

The carbon nanotubes generated for use in connection with the present invention may be provided with certain characteristics. In accordance with one embodiment, diameters of the carbon nanotubes generated may be related to the size of the catalyst particles. In particular, the diameters for single wall nanotubes may typically range from 0.5 nanometers (nm) to 2 nm or more for single wall nanotubes, and from 2 nm up to 50 nm or more for multi-wall nanotubes. In addition, it should be noted that the nature of these carbon nanotubes, for instance, their metallic or semiconductor character, may correspond to their diameter, their chirality and/or their defects, if any. Accordingly, in order to control the nature or characteristic of these nanotubes, it may be necessary to control their dimensions with sufficient accuracy.

Moreover, the strength of the SWNT and MWNT generated for use in connection with the present invention may be 30 GPa maximum. Strength, as should be noted, can be sensitive to defects. Nevertheless, the elastic modulus of the SWNT and MWNT fabricated for use with the present invention is typically not sensitive to defects and can vary from 1 to 1.5 TPa. Moreover, the strain to failure, which generally can be a structure sensitive parameter, may range from a few percent to a maximum of 10% in the present invention.

These parameters and characteristics, when taken together, suggest that the carbon nanotubes produced by methods of the present invention can be a sufficiently strong material for use in the subsequently production of a carbon composite.

### Carbon-Carbon Composite

The present invention provides, in one embodiment, a process for manufacturing a carbon-carbon composite from at least one sheet of non- woven carbon nanotubes or nanofibers (i.e., carbon nanotube paper).

The sheets of non- woven fibers, in an embodiment, may be made by initially harvesting carbon nanotubes made in accordance with a CVD process as disclosed in U.S. Patent Application Publication US 2005/0170089, which application is hereby incorporated herein by reference.

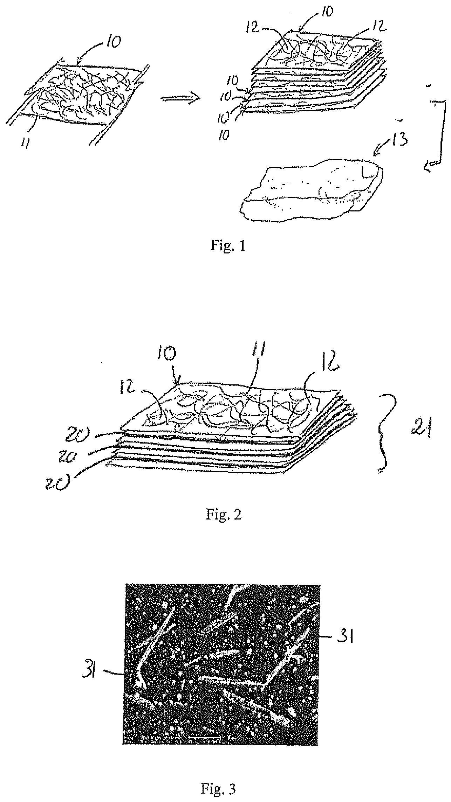

Looking now at Fig. 1, the harvested carbon nanotubes 11 may thereafter be layered in a non-woven, overlapping manner in the presence of a binder material to form a sheet 10, similar to the process for making paper. Alternatively, the nanotubes 11 may be wound into fibers and the fibers layered in a non-woven, overlapping manner in the presence of a binder material to form sheet 10. Examples of a suitable binder material includes any thermoplastic material including, for example, polyolefins such as polyethylene, polypropylene, polybutene-1, and poly-4-methyl-pentene-1; polyvinyls such as polyvinyl chloride, polyvinyl fluoride, and polyvinylidene chloride; polyvinyl esters such as polyvinyl acetate, polyvinyl propionate, and polyvinyl pyrrolidone; polyvinyl ethers; polyvinyl sulfates; polyvinyl phosphates; polyvinyl amines; polyoxidiazoles; polytriazols; polycarbodiimides; copolymers and block interpolymers such as ethylene-vinyl acetate copolymers; polysulfones; polycarbonates; polyethers such as polyethylene oxide, polymethylene oxide, and polypropylene oxide; polyarylene oxides; polyesters, including polyarylates such as polyethylene terphthalate, polyimides, and variations on these and other polymers having substituted groups such as hydroxyl, halogen, lower alkyl groups, lower alkoxy groups, monocyclic aryl groups, and the like, and other thermoplastic meltable solid materials.

Alternatively, the sheets of non-woven carbon nanotubes may be obtained from any commercially available source.

Next, a plurality of non- woven sheets 10 may be next be layered on one another and, in one embodiment, be provided with at least one coating of a resin material, such as furfuryl alcohol (C₅H₆O₂). The coating of resin material can infiltrate voids 12 between the overlapping carbon nanotubes 11, and subsequently provide structural integrity to the resulting composite material. The amount of furfuryl alcohol used may be determined in accordance with the amount of carbon nanotubes 11 in the non- woven sheet 10. In particular, the ratio of carbon from the furfuryl alcohol to the carbon in the nanotubes 11 can range, in an embodiment, from 1:1 to 10:1. In an embodiment where a substantially non-porous composite may be generated, a ratio of carbon from the furfuryl alcohol to the carbon in the nanotube 11 may be 3:1.

It should be noted that coating of the non-woven sheets 10 can be performed on each individual sheet 10 prior to the sheets 10 being layered on one another. Moreover, if desired, prior to infiltrating the voids with a resin material, a surface treatment process can be applied to the carbon nanotubes to facilitate wetting (i.e., bonding) of the resin material to the nanotubes. Such surface treatment can be implemented by methods well known in the art.

The coating of furfuryl alcohol on the sheets 10 of non-woven carbon nanotubes 11 may then be allowed to evaporate and polymerize with the nanotubes 11 at a temperature ranging from 50° C to 150° C. To the extent that the resin material may be available in a polymerized formed, exposure to heat for polymerization may not be necessary.

Thereafter, the coated sheets 10 may be hot pressed to bond the sheets of non-woven carbon nanotubes with one another into a formed mass or structure 13. The pressing, in one embodiment, may be done at a temperature range of from 125° C to 350° C, and at a pressure of at least 3000 psi for approximately 10 minutes or until the sheets 10 are bonded to one another. It should be appreciated that the temperature, pressure and length of time can be dependent of the type of resin selected.

In an alternative embodiment, with reference now to Fig. 2, a thin sheet of a polymeric resin, such as RESOL resin, polyamide, epoxy, Krayton, polyethylene, or PEEK (polyaryletherketone) resin, other commercially available resins, or a combination thereof, may be positioned between adjacent sheets 10 of non-woven carbon nanotubes 11.

This sandwich structure 21 of non- woven sheets 10 and resin 20 may then be hot pressed to bond the sheets 10 of non-woven carbon nanotubes 11 with one another into a form. The pressing, in one embodiment, may be done at a temperature range of from 125° C to 350° C, and at a pressure of at least 3000 psi for approximately 10 minutes or until bonding of the sheets occurs. By pressing in such a manner, the sheets 20 of polymeric resin may soften and flow to infiltrate voids 12 between overlapping carbon nanotubes 11 within each non- woven sheet 10, and permit the non- woven sheets 10 to bond with one another to provide a formed mass or structure 13. Again, the temperature, pressure and length of time can be dependent of the type of resin selected.

It should be appreciated that, similar to the coating approach, if desired, prior to infiltrating the voids with a resin material, a surface treatment process can be applied to the carbon nanotubes to facilitate bonding of the resin material to the nanotubes. Such surface treatment, again, can be implemented by methods well known in the art.

Once bonded, the sheets 10 of non-woven carbon nanotubes 11 in formed mass 13 may be subject to pyrolysis for curing. In particular, the formed structure 13 may be subject to slowly increasing temperature, for instance, less than 1 degree C per minute. In an embodiment, the curing temperature may be raised to at least between 1000° C and 2000° C, and more preferably 1700° C to form a carbon-carbon composite. This slow heating rate, in one embodiment, allows water, a primary fluid by-product of the reaction, to diffuse out of the formed structure 13 and permits the structure 13 to be cured into the carbon-carbon composite.

To the extent desired, this cured or paralyzed carbon-carbon composite may be hot pressed over or into a mold having a shape of a final product or structure, and may be further pyrolyzed for final curing. Specifically, the composite may be subject to a final ramp temperature up to 3000° C to anneal (i.e., remove any defects) the composite in the shape of the desired product or structure.

Although reference is made to the use of multiple sheets 10 of non- woven carbon nanotubes 11, it should be appreciated that the only one non- woven sheet 10 may be used in the manufacturing of a carbon-carbon composite. The number of sheets 10 employed may be dependent on the desired percentage weight of carbon nanotubes per unit area of the composite to be manufactured. In other words, to obtain a relatively high percentage weight of carbon nanotubes per unit area, additional sheets 10 of non-woven carbon nanotubes 11 are used.

Moreover, the thickness of each non-woven sheet 10, in an embodiment, can range from 0.01 mm up to more than 1 cm. It should be appreciated that curing may need to take into account the time for reaction products to diffuse out of the structure 13. Since the primary reaction product during this process is a fluid, such as water, the amount of time necessary for curing can be significant for thicknesses of more than 3 mm.

One method of increasing thickness of each non-woven sheet 10 beyond 3 mm may be to coat each layer (i.e., sheet) with a diluted resin on a heated substrate, so that curing of the resin can occur before the next layer 10 may be applied. Another method of making thicker sheets and thus composites may be to provide channels within the non-woven sheets 10 to allow reaction products (e.g., water) to escape more easily.

It should be noted that in the embodiment where thin sheets 20 of polymeric resin may be used, resin sheets of different polymers may be positioned between different adjacent non-woven sheets 10 to create a structure or device, such as a protective helmet, with different properties on the outer surface than in the interior. For example a pigmented polymer resin layer may be placed on the outer surface of the composite to eliminate painting.

Alternatively, a polymer resin layer containing a roughening element, such as walnut shell fragments presently used in military helmets, can be incorporated in one molding step. Other types of gradients in properties may also be advantageous to help distribute impact energy from a projectile over a larger volume element. This may be done by layering the non-woven carbon nanotube sheets 10 close together towards the outer layers and spreading them out towards the interior layers, and/or by changing the type of binder used as a function of the thickness.

Moreover, structures or devices made from the composite manufactured in accordance with this process of the present invention can maintain their properties at elevated temperatures. For example, with PEEK resin, the composite can be expected to maintain its strengths and properties at temperatures of 160° C and usable at temperatures of up to 260° C.

### Glassy Carbon Matrix Composite

In accordance with another embodiment that is not according to the present invention, there is provided a process for generating a composite material having a glassy carbon matrix reinforced by a "grown-in" array of carbon nanotubes. In other words, the process provides an approach wherein an array of nanotubes may be permitted to form and grow by solid state transport within a carbon containing resin material, which resin material may subsequently be transformed into a glassy carbon matrix composite.

In particular, the process includes initially adding a suitable catalyst to a carbon-containing resin. Examples of a suitable catalyst include, ferrocene, iron nano-particles, iron pentacarbonyl, nano-particles of magnetic transition metals, such as, cobalt, cobalt hexacarbonyl, nickel, nickel hexacarbonyl, molybdenum or their alloys, or oxides, nitrates or chlorides of these metals or any combination of the oxides or other reducible salts (e.g., iron ammonium sulfate or iron chloride) or organometallic compounds of these metals. Examples of a suitable carbon-containing resins for use in the present process include a high- carbon-containing resin, such as RESOL resin (i.e., catalyzed alkyl-phenyl formaldehyde), which can be obtained from Georgia Pacific, furfuryl alcohol, or pitch.

The catalyst particles, in an embodiment, may be added at an appropriate concentration to the carbon-containing resin, so as to provide the resulting composite material with optimal properties. To that end, the concentration of the catalyst particles used in connection with the present invention may be a function of concentration of carbon in the resin. In an example where ferrocene (Fe(C₅H₅)₂) is added to high-carbon-containing RESOL phenyl formaldehyde, the concentration of ferrocene may range from 0.005 percent to 5 percent by weight. More particularly, the ratio of ferrocene may be 2 percent by weight (iron to carbon). Alternatively, the catalyst particles may be substantially uniformly dispersed throughout the resin to provide an appropriate concentration.

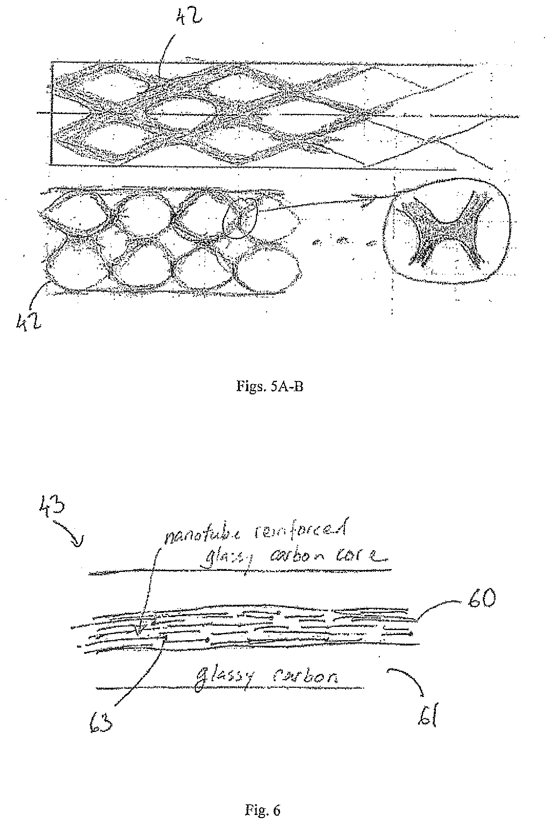

Next, the high-carbon-containing resin having the catalyst particles dispersed therein may be subject to pyrolysis for curing. In particular, the resin imbedded with the catalyst particles may undergo a slow increase in temperature, for instance, less than 1 degree C per minute, in an inert atmosphere, such as argon, or helium. In an embodiment, the temperature may be raised to at least between 1000° C and 2000° C, and more preferably 1700° C. This slow increase in temperature, in one embodiment, allows water, the primary by-product of the reaction, to diffuse out of the resin material. In addition, the catalyst material, such as ferrocene, in the presence of high temperature, breaks down and forms, for instance, particles of iron which can act as a template to which carbon within the high-carbon- containing resin can attach. The attachment of carbon to the template particles and the subsequent attachment to the existing carbon on the template particles occurs in series, so as to lead to the growth of a nanotube from a particle, and the formation of an array of carbon nanotubes within the resin. The result can be the formation of a composite material having a glassy carbon matrix reinforced by a "grown-in" array of carbon nanotubes. As illustrated in Fig. 3, scanning electron micrographs of the surface of a composite material manufactured in accordance with this embodiment of the invention show the presence of an array of multiwall carbon nanotubes 31. In an embodiment, the process can generated substantially aligned nanotubes (see Fig. 6).

To the extent necessary, the activity of the catalyst particles (e.g., iron particles) may need to be augmented. In one embodiment of the invention, thiophene (C₄H₄S) or another sulfur containing compound, for example, may be added to the resin prior to or during pyrolysis to augment the activity of the catalyst particles. In addition, it may be desirable to add trace amount of, for instance, Nb, Mo, Cr, or a combination thereof to the resin prior to or during pyrolysis to refine the size of the catalyst particles, in order to control the size of the nanotubes being grown.

Moreover, if desired, the glassy carbon matrix composite may be exposed to a final ramp temperature up to 3000° C to anneal the composite to remove any potential defects within the composite.

It should be noted that although carbon nanotubes are disclosed herein, the present process may be implemented in a manner which includes chemically modifying the carbon in whole or in part, or by replacing the carbon with, for instance, silicon, boron or nitrogen, so that nanotubes can be generated containing elements other than or in addition to carbon. For instance, the nanotubes may be silicon-carbon nanotubes, boron-carbon nanotubes, nitrogen-carbon nanotubes, or a combination thereof.

The in situ composite having a glassy carbon matrix reinforced by a "grown-in" array of carbon nanotubes created in accordance with the above process may have a wide variety of applications based not only on mechanical properties, but also on chemical and electrical properties. Unlike other types of fiber composites, this type of in situ composite, for instance, can be cast into complex three-dimensional shapes or structures, coated on a substrate, provided as a thin film, or extruded as a filamentous fiber, then subsequently paralyzed to form the desired structure or coating fiber. It should be noted that liquid viscosity would not be substantially changed, since the nanotubes are grown within the structure after polymerization, followed by pyrolyzation.

For extrusion as a filamentous fiber, in one embodiment, the catalyzed resin may be extruded through a nozzle having an aperture at a temperature ranging from 50° C to 150° C to polymerize an exiting monofilament or fiber. In accordance with an embodiment, the nozzle or aperture may be provided with a diameter of from 0.5 microns to 500 microns to extrude a mono-filament of similar size. Upon subsequent pyrolization at temperature ranging from 1000° C to 2000° C, followed by a ramp up temperature of up to 3000° C, the extruded monofilament can be converted into a filament of glassy carbon with a substantial amount of carbon nanotubes along its length.

In addition, properties of the structure, coating or extrusion formed from this in situ composite can be tailored by changing the catalyst concentration or material within the composite, coating thin film, or filament. For example, silicon may be added to the outer portions of a structure, so as to form an oxidation-resistant coating of silicon carbide upon pyrolyzation. These capabilities can lead to the creation of devices, such as heart valves or blood vessel stents, as well as components (i.e., parts) of a device, including medical and surgical device. These devices or components, in one embodiment, may be provided with nanotubes in the high strength area, since nanotubes are known to have relatively high strength, and pure glassy carbon matrix in areas subject to harsh chemical environments or in areas where biocompatibility can be important, since glassy carbon matrix can withstand such environments.

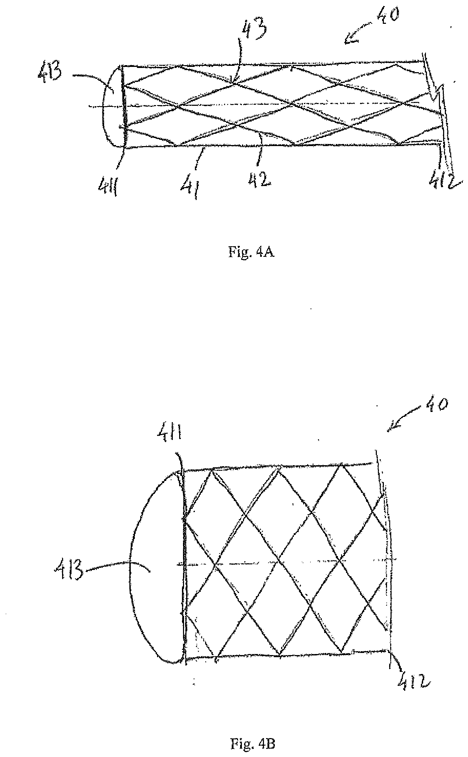

For example, a RESOL resin, catalyzed as described above, may initially be placed in a reusable mold designed to produce an embossed pattern of stent 40 illustrated in Figure 4. This mold, in an embodiment, may be formed in two parts and can be made of silicon rubber. One part, the interior mold, can contain the pattern, while the other part, the exterior mold, can hold the resin. The mold may then be placed in a vacuum chamber, and evacuated to a rough vacuum. Thereafter, the resin may be placed in the mold, in a manner well know in the art of casting. The mold and resin may next be very slowly heated to the temperature of 150° C, at which the resin polymerizes, and subsequently allowed to cool to form a glassy carbon precursor. The now polymerized glassy carbon precursor may thereafter be carefully removed from the mold, and reheated at a temperature range of from 1000° C to 2000° C at a rate of less than 1 degree per minute to form the desired structure. It should be appreciated that a faster rate of increase in temperature may be possible since this type of structure can be thin and water may diffuse out of the structure rapidly. Other parts or devices can be cast in a similar manner.

In an alternate embodiment where a relatively thicker structure may be necessary, the high-carbon-containing resin may be provided as an aerosol. The high-carbon-containing resin aerosol may be sprayed, for instance, in the presence of a catalyst, onto a hot substrate. In the presence of heat from the substrate, formation and growth of carbon nanotubes, in the manner noted above, can be initiated. Such an approach would allow the build up of thicker, substantially more uniform components or devices, especially when the substrate may be rotating and the component being manufactured needs to be centro-symmetric.

### Applications

The composite material generated from either of the processes above, in accordance with one embodiment of the present invention, may be provided with more than 5% carbon nanotubes by weight and may be utilized in a variety of applications.

Referring now to Figs. 4A-B, there is shown a stent 40 made from the composite materials described above. The stent 40, in an embodiment, includes a substantially tubular body 41 having a pathway 413 extending between ends 411 and 412. The tubular body 41, in one embodiment, may be made to include an expandable matrix 42 having intersecting filaments 43 to permit stent 40 to transition between an elongated or collapsed state (Fig.4A) prior to insertion into an artery or vessel (e.g., a blood vessel) and an expanded state (Fig. 4B) subsequent to insertion into the artery or vessel. It should be appreciated that matrix 42 may be patterned in any number of ways, so long as it permits tubular body 41 to move between a collapsed state and an expanded state insertion. Examples of matrix patterns that may be employed are illustrated in Figs. 5A and 5B.

Since stent 40 may be made from the composite materials described above, looking now at Fig. 6, each filament 43, in an embodiment, may be provided with carbon nanotubes 60 towards the interior of filament 43, so as to provide stent 40 with sufficient strength. In addition, each filament 43 may be provided with glassy carbon material 61 the nanotubes 60 towards the exterior of filament 43 to permit interaction with fluid within the vessel. Since pathway 42 of tubular body 41 may be defined by the exterior of filaments 43, the presence of the biocompatible glassy carbon material thereat permits pathway 42 to interact with the fluid within the vessel in a biocompatible manner.

Furthermore, since each of the provided nanotubes 60 may be grown from a catalyst particle, such as an iron catalyst, each nanotube 60 may include a catalyst particle 63 at one end, that is, the end from which growth was initiated. The presence of the catalyst particles 63 within the interior of filaments 43 can allow stent 40 to be visible, for instance, in an x-ray to permit a high degree of accuracy when locating or placing the stent 40 within the vessel. To further enhance visibility of stent 40, additional contrast agents can easily be added within the interior of filaments 43. Examples of contrasting agents that may be used include BaO, TaO₂, WO₃, HfO, WC, Au nano or micro-powders, or a combination thereof. In this application, the presence of iron catalysts can also serve to provide the stent 40 with magnetic properties. Magnetic properties, of course, can be imparted when catalysts with magnetic properties are used.

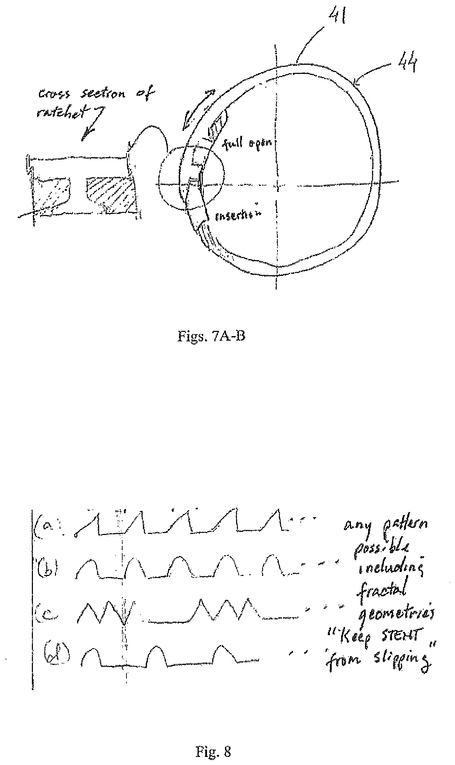

Tubular body 41, in an embodiment, may further include an exterior surface 44 that can be patterned. By providing tubular body 41 with a patterned exterior surface 44, movement of stent 40 along or within a vessel may be minimized, since the patterned surface 44 may act to anchor tubular body 41 against a surface of the vessel. It should be noted that a variety of patterns on the exterior surface 44 may be employed in connection with tubular body 41, so long as such patterns can minimize or prevent movement of stent 40 within the vessel. Figs. 7-8 illustrate various patterned designs that may be employed in connection with the exterior surface 44, including a ratchet pattern (Figs. 7A-B), or other patterns, for instance, etched lines, swirls or any other geometric shapes, such as those illustrated in Fig. 8.

Expansion of the tubular body 41 of stent 40 may be accomplished by any methods known in the art, for instance, a balloon device, or by the use of shape-memory technology that can allow the tubular body 41 to automatically expand subsequent to insertion into the vessel. Moreover, expansion of stent 40 can further permit the patterned exterior surface 44 of stent 40 to better engage interior walls of the vessel within which the stent 40 may be placed to hold and maintain the stent 40 in place. In an embodiment, the pressure exerted radially by the geometry of stent 40 may be governed by the holding power of the patterned exterior surface 44 as well as the elastic properties of the composite.

It should be noted that although reference is made to a stent, other biocompatible and implantable biomedical devices may be made using the composite materials set forth in the present invention. For instance, anchoring screws for use in ACL and PCL reconstruction and other orthopedic implants.

The processes illustrated above, along with the composite material generated therefrom, can be utilized for other applications or manufacturing of other devices. For example, the process may be used to form ballistic armor. In one embodiment, the ballistic armor may be formed by initially coupling or layering commercially available body armor textile fabric or textile made from carbon nanotubes, yarns created from the carbon nanotubes, or carbon nanotube webs or belts. Next, a catalyzed resin, as described above, may be used to coat or couple a plurality of body armor textile fabric sheets and hold the sheets together, so as to contribute to the strength of the armor, as well as help to minimize or prevent cracks in the armor. The coated sheets may be pyrolyzed to generate the end product.

Other applications may include: (1) molded high strength parts, such as combat helmets, motorcycle helmets, football helmets and the like, (2) aerospace parts being used for high temperature applications, such as leading edges for hypersonic use, rocket nozzles etc., (3) motor parts, such as brake pads and bearings, (4) sporting goods, such as rackets, golf clubs, bicycle frames, (5) parts for use at substantially high temperature, including thermal conductors, electrical conductors, structural lightweight panels, and coatings for graphite, so as to reduce cost, while maintaining a high strength wear resistant surface, (6) engraving plates made from glassy carbon that can be highly resistant to wear and corrosive properties of inks used in intaglio or other forms of printing, and (7) biocompatible implantable parts or components, such as heart valves and stents, graded so that the glassy carbon matrix comes substantially into contact with body fluids, while the nanotube portions can be located in center regions or core areas of the composite and in high stress areas.
While the invention has been described in connection with the specific embodiments thereof, it will be understood that it is capable of further modification.

## Claims

1. A composite material comprising:
a mass having a thickness ranging from 0.01 mm to more than 3 mm, the mass formed by a plurality of non-woven nanotube sheets bonded to one another;
a plurality of voids between the non-woven nanotubes; and
a resin material situated within the voids between the non-woven nanotubes to provide the mass with structural integrity.

2. A composite material as set forth in claim 1, further including channels extending throughout the mass, the channels providing a pathway to permit fluid byproducts to escape during manufacturing of the composite material.

3. A composite material as set forth in claim 1, wherein the non- woven nanotubes exist in an amount that is more than 5 % by volume of the mass.

4. A composite material as set forth in claim 1, wherein the nanotubes include one of carbon nanotubes, silicon-carbon nanotubes, boron-carbon nanotubes, nitrogen- carbon nanotubes, or a combination thereof.

5. A composite material as set forth in claim 1, wherein the resin material in one area of the mass is different than the resin material in another area of the mass, so as to provide different properties in those areas.

6. A composite material as set forth in claim 1, wherein the mass is capable of being formed into a three dimensional shape or structure.

7. A composite material as set forth in claim 6, wherein the three dimensional shape or structure includes molded high strength parts, including combat helmets, motorcycle helmets, football helmets and the like, parts for high temperature applications, including hypersonic parts and rocket nozzles, and biomedical devices and parts, including hear valves and stents. A composite material is described. The composite material comprises a mass having a thickness ranging from about 0.1 mm to more than about 3mm. The composite also includes a plurality of non-woven nanotubes dispersed throughout the mass, such that a plurality of voids exist between the nanotubes. The composite further includes a resin material situated within the voids between the non-woven nanotubes to provide the mass with structural integrity.

## Patentansprüche

1. Verbundmaterial, umfassend:
eine Masse mit einer Dicke im Bereich von 0,01 mm bis mehr als 3 mm, wobei die Masse aus einer Vielzahl von miteinander gebondeter Nanoröhrenvliesplatten gebildet ist;
eine Vielzahl von Hohlräumen zwischen den Vlies-Nanoröhren; und
ein in den Hohlräumen zwischen den Vlies-Nanoröhren angeordnetes Harzmaterial, um der Masse Standsicherheit zu verleihen.

2. Verbundmaterial nach Anspruch 1, weiter enthaltend Kanäle, die sich durch die Masse erstrecken, wobei die Kanäle einen Weg bereitstellen, um Fluidnebenprodukten zu erlauben, während des Herstellens des Verbundmaterials zu entweichen.

3. Verbundmaterial nach Anspruch 1, wobei die Vlies-Nanoröhren in einer Menge vorliegen, die mehr als 5 Volumen-% der Masse ist.

4. Verbundmaterial nach Anspruch 1, wobei die Nanoröhren eines von Kohlenstoff-Nanoröhren, Silizium-Kohlenstoff-Nanoröhren, Bor-Kohlenstoff-Nanoröhren, Stickstoff-Kohlenstoff-Nanoröhren oder eine Kombination davon enthalten.

5. Verbundmaterial nach Anspruch 1, wobei das Harzmaterial in einem Gebiet der Masse anders ist als das Harzmaterial in einem anderen Gebiet der Masse, sodass unterschiedliche Eigenschaften in jenen Gebieten bereitgestellt werden.

6. Verbundmaterial nach Anspruch 1, wobei die Masse zu einer dreidimensionalen Gestalt oder Struktur geformt werden kann.

7. Verbundmaterial nach Anspruch 6, wobei die dreidimensionale Gestalt oder Struktur geformte hochfeste Teile, umfassend Schutzhelme, Motorradhelme, Footballhelme und dergleichen, Teile für Hochtemperaturanwendungen, umfassend hypersonische Teile und Raketendüsen und biomedizinische Vorrichtungen und Teile, umfassend Hörventile und Stents, beinhaltet.

## Revendications

1. Matériau composite comprenant:
une masse ayant une épaisseur allant de 0,01 mm à plus de 3 mm; lamasse formée par une pluralité de nanotubes non tissés liés entre eux,
une pluralité de vides entre les nanotubes non tissés; et
un matériau de résine situé à l'intérieur des vides entre les nanotubes non tissés pour conférer à la masse une intégrité structurale.

2. Matériau composite selon la revendication 1, incluant en outre des canaux s'étendant dans la masse, les canaux fournissant une voie pour permettre aux sous-produits fluides de s'échapper pendant la fabrication du matériau composite.

3. Matériau composite selon la revendication 1, dans lequel les nanotubes non tissés existent en une quantité supérieure à 5 % en volume de la masse.

4. Matériau composite selon la revendication 1, dans lequel les nanotubes incluent l'un des nanotubes de carbone, des nanotubes de silicium-carbone, des nanotubes de bore-carbone, des nanotubes d'azote-carbone, ou une combinaison de ceux-ci.

5. Matériau composite selon la revendication 1, dans lequel le matériau de résine dans une zone de la masse est différent du matériau de résine dans une autre zone de la masse, de manière à fournir des propriétés différentes dans ces zones.

6. Matériau composite selon la revendication 1, dans lequel la masse est susceptible d'être formée en une forme ou structure tridimensionnelle.

7. Matériau composite selon la revendication 6, dans lequel la forme ou structure tridimensionnelle inclut des pièces moulées de haute résistance, y compris des casquesde combat, descasquesde moto, descasquesde football et similaires, despièces pour des applications à haute température, incluant des pièces hypersoniques et des tuyères de fusée; et des dispositifs et pièces biomédicaux, incluant des valves auditives et des stents.
